(19)

Europäisches
Patentamt
European
Patent Office
Office européen
des brevets

(11) **EP 1 854 906 A2**

(12) **EUROPEAN PATENT APPLICATION**

(43) Date of publication:
**14.11.2007 Bulletin 2007/46**

(51) Int Cl.:
***C23C 16/44*** *(2006.01)*

(21) Application number: **07251849.1**

(22) Date of filing: **02.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **09.05.2006 US 430614**

(71) Applicant: **Air Products and Chemicals, Inc.**
**Allentown, PA 18195-1501 (US)**

(72) Inventors:
• **Maroulis, Peter James**
  **Alburtis**
  **PA 18011 (US)**

• **Ketkar, Suhas Narayan**
  **Allentown**
  **PA 18104 (US)**
• **McDermott, Wayne Thomas**
  **Fogelsville**
  **PA 18051 (US)**

(74) Representative: **Muir, Benjamin M. J.**
**Beck Greener**
**Fulwood House**
**12 Fuldwood Place**
**London WC1V 6HR (GB)**

(54) **Analysis of a reactive gas such as silane for particle generating impurities**

(57)     A process, for supplying to a processing tool such as a chemical vapor deposition device a gas that may contain an impurity that reacts and/or nucleates to form contaminating particles suspended in the gas, comprises sampling a flow (25) of the gas upstream of the processing apparatus with a particle counter (50) and/or particle capture filter (60) to detect an amount of the contaminating particles in the flow of the gas, and generating a signal when the amount of particles is in excess of a predetermined amount. The process may, in particular, be an integrated circuit manufacturing process, wherein the gas is silane.

FIG. 3

EP 1 854 906 A2

## Description

BACKGROUND OF THE INVENTION

The present invention pertains to a system and method for the detection of molecular contaminants in a matrix gas, and particularly to the detection of impurities capable of reacting and/or nucleating to form particles suspended in a matrix gas. Examples of such contaminants include oxygen, water, certain hydrocarbons and siloxane impurities. An example of a matrix gas is silane ($SiH_4$) used for semiconductor manufacturing processes.

Semiconductor device manufacturers use process gases such as silane in critical applications such as thin silicon and silicon oxide film deposition. Such thin films must be substantially free of contaminating impurities and impurity generated particulate material. Any trace impurities or impurity generated particles tend to form defects, possibly resulting in failure, performance reduction, or reliability problems in microelectronic devices. Especially troublesome impurities include water, oxygen, certain hydrocarbons and siloxanes. These impurities have been found to lower thin film quality in devices having ever-shrinking feature sizes. Water and oxygen are highly abundant atmospheric substances that can readily enter silane production systems, containers and gas distribution systems. Hydrocarbons and siloxanes can readily contaminate silane during its production and storage.

Water is especially difficult to completely remove from ultra-high purity (UHP) gas systems due to its polar nature. Polar water molecules tend to remain adsorbed on surfaces that have been exposed to moist atmospheres. Typical methods for removing water from UHP gas systems include prolonged heating, combined with pressure cycling, using high purity flush gas and vacuum. Nevertheless, physically adsorbed and chemically adsorbed water (or moisture), present in trace quantities, can only be removed with great difficulty from contaminated surfaces.

Non-polar oxygen molecules can be removed with comparatively less difficulty from UHP systems, but can pose special problems in silane systems. Oxygen is highly abundant in the atmosphere, and can therefore easily penetrate inward to pressurized gas systems. The high partial pressure of oxygen in the atmosphere provides a strong driving force for diffusion to even pressurized gas systems. Such entry can occur through even microscopic leak points in the system. High concentration gradients also allow oxygen and water to enter UHP gas systems via permeation through porous materials.

Both water and oxygen react with silane to form siloxanes and solid (particulate) silica. The oxygen in silica can be derived from water, oxygen or other oxygen-containing contaminants. Unreacted water can exist in silane at concentrations as high as 1 ppm. However, oxygen and water have also been found to react readily with silane at concentrations below 1 ppm to form particulate matter. Particles larger than 0.25 micrometer have been detected at these impurity concentrations. Particle concentrations were found to increase monotonically with the concentration of water or oxygen. Reaction between water or oxygen and silane at lower concentrations may also be possible. Water reacts less readily than oxygen with silane in the gas stream. However, the reactivity of water with silane increases in the presence of a surface, such as tubing, other system components, or a catalyst. Water, oxygen and their reaction products are all damaging to integrated circuit (IC) devices during fabrication.

Contaminating impurities may thus generate contaminating particles through a first mode of particle formation consisting of chemical reaction with the matrix gas. Contaminating impurities may also generate contaminating particles through a second mode of particle formation consisting of nucleation during pressure reduction.

Many semiconductor processing gases are supplied in pressurized vessels. It is common for such high purity gases to contain trace molecular impurities, such as, for example, hydrocarbons in nitrogen, hydrocarbons and/or siloxanes in silane, and other such impurities depending upon the composition of the UHP gas. These impurities may result from the processes used to produce, transfer and store the gases in pressurized containers. The internal pressure and temperature of the gas storage vessel are frequently well above the critical point pressure and critical point temperature of the gas. For example, the critical points of nitrogen (492 psia, -232°F; 3.39 MPa, -147°C) and silane (703 psia, 26°F; 4.85 MPa, -3°C) are typically exceeded in gas storage vessels as delivered to users. It is well-known that supercritical fluids have a high solvent power for contaminating materials, such as higher molecular weight hydrocarbons, which may exist as surface contaminants in gas transfer, storage and delivery systems. These dissolved impurities may add to the molecular impurities typically present in the gas.

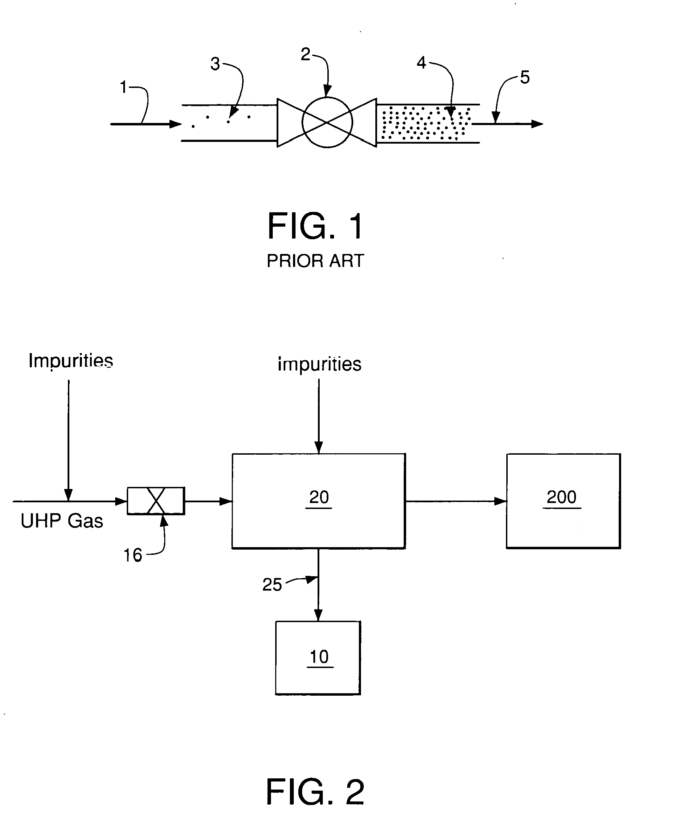

It is well-known in the art of particle measurement that gases having trace quantities of molecular impurities can suffer an increase in particle content as the gas pressure is reduced. See, e.g., Wen et al., "Nucleation of Trace Amounts of Condensible Vapors in an Expanding Gas Jet." J. Aerosol Sci., Vol. 19, No. 1, 153-156 (1988). This increase results from molecular clustering of trace impurities leading to formation of stable (i.e., persistent) suspended particles. These impurity generated particles cannot be easily vaporized through heating. Further, in certain instances, the process of pressure reduction frequently produces sub-critical conditions in the gas. In this regard, the sub-critical gas loses its high solvent power following pressure reduction. Any dissolved impurities therefore tend to form stable suspended particles in the lower pressure gas stream. Particle formation during pressure reduction is known to produce particle levels of over $10^6$ per standard cubic foot (28 standard liters; "28 NL"); of gas for particles larger than 0.02 micrometer. This level substantially exceeds the original level of particles in the pressurized gas. Fig. 1 provides an example of a typical gas feed stream 1 that is passed through a pressure reducing device 2 such as a valve, an automatic pressure regulator, a flow-restricting orifice, or the like, that is in fluid communication with gas feed stream 1. While the gas feed stream initially contains low levels of gas-borne particles 3, after passing through pressure reducing device 2, the amount of particles contained within gas feed stream 1 or "nucleated" particles 4 increases. These nucleated particles 4 within lower pressure gas stream 5 are carried to downstream processing equipment (not shown).

System contamination is frequently detected as failure of IC devices following complete fabrication. Such failures are costly to the semiconductor industry. Accordingly, any UHP gas system upset or other deviation resulting in increased levels of contamination must be rapidly and accurately detected through continuous monitoring. For example, any gas system upset resulting in increased oxygen, water, hydrocarbon or siloxane levels must be detected and remedied Prior to significant IC device loss. Only through such monitoring can IC product loss be minimized. Control of water, oxygen, hydrocarbons and siloxanes in UHP silane therefore requires sensitive methods for continuous or intermittent monitoring of trace impurities levels.

Ever shrinking device sizes are sensitive to ever lower levels of impurities and impurity generated particles. Impurities monitoring instruments, such as atmospheric pressure ionization mass spectrometry (APIMS), have lower detection limits ($LDL_S$) of less than 100 parts per trillion (ppt). However, such instruments require careful calibration with accurate standards, and are costly to purchase, operate and maintain. State-of-the-art purification technology capable of producing low ppt reference gas is necessary to establish the limit of detection. The detection limit is highly sensitive to such factors as instrument bake-out temperature and bake-out time. Moreover, a specialized APIMS utilizing a bi-compartment source is needed to ensure that the presence of silane in the corona discharge does not plate the discharge needle with silicon-containing compounds.

In addition, such molecular impurities monitors cannot detect water or oxygen that has already reacted with silane to form particulate silica. Therefore, leaks, residual molecular impurities, or other such system deviations may not be detected by molecular impurities monitors.

Accordingly, it is desired to provide means for detecting impurities and impurity generated particles in various UHP processing gases. it is further desired to provide such means capable of detecting impurities present at levels as low as 100 ppt, more preferably at levels as low as 10 ppt, still more preferably at levels as low as 1 ppt, which means do not suffer from one or more of the aforementioned deficiencies of APIMS.

BRIEF SUMMARY OF THE INVENTION

Accordingly, a first aspect of the invention comprises an improved process for supplying to a processing apparatus a gas containing at least one impurity that reacts and/or nucleates to form contaminating particles suspended in the gas. The improved process comprises sampling a flow of the gas upstream of the processing apparatus with a particle counter and/or particle capture filter to detect an amount of the contaminating particles suspended in the flow of the gas, and generating a signal when the amount of contaminating particles is in excess of a predetermined amount.

A second aspect of the invention comprises an improved process for manufacturing integrated circuits in which gaseous silane is supplied to a processing apparatus. The improved process comprises sampling a flow of the silane upstream of the processing apparatus with a particle counter and/or particle capture filter to detect an amount of contaminating particles suspended in the flow of the silane, and generating a signal when the amount of contaminating particles is in excess of a predetermined amount.

A third aspect of the invention comprises an apparatus for maintaining a purity level of a gas feed stream of a processing apparatus at or above a minimum acceptable purity level, said apparatus comprising: a supply source for supplying the gas; at least one processing tool in fluid communication with the supply source and adapted to use the gas to perform a processing function; a particle counter placed upstream of the processing tool and downstream of the supply source; a microprocessor in electrical communication with at least the particle counter; and optionally a particle capture filter arranged in parallel with the particle counter upstream of the processing tool and downstream of the supply source.

BRIEF DESCRIPTION OF SEVERAL VIEWS OF THE DRAWINGS

The invention will be described in conjunction with the following drawings in which like reference numerals designate like elements and wherein:

Fig. 1 is a schematic view of a prior art pressure reducing device; and

Figs. 2 and 3 are flow charts depicting embodiments of the inventive system and method.

DETAILED DESCRIPTION OF THE INVENTION

The invention comprises a system and method for detecting minute quantities of molecular impurities in gas streams by measurement of suspended particulate reaction products of the contamination. The invention further comprises a system and method for detecting minute quantities of moiecuiar impurities in gas streams by first inducing said impurities to nucleate into suspended particles in a process of pressure reduction, then measuring said suspended particles.

UHP gas system molecular contamination is often first evident as a presence of microscopic particles such as silica. Some particles may be present as surface contamination of the UHP system. However, the particles are often suspended in the flowing gas stream and carried along with the gas. The preferred embodiment of the invention comprises a method for detecting minute quantities of reacting or nucleating molecular contamination of UHP gases using an automatic particle counter and/or particle capture filter. The size and number concentration of particles detected by the particle counter and/or capture filter are used to determine the level of impurities originally present in the gas. In one embodiment of the invention, reactive molecular impurities are detected following spontaneous formation of particles in the gas system. In another embodiment of the invention "nucleating" molecular impurities are detected following induced formation of particles through pressure reduction of a sample gas stream.

A system and method that can be used for the measurement and/or analysis of particles within a gas stream is described herein. The system and method may be used to determine, for example, the number of particles or particle count; the concentration density of particles within the gas stream; the particle size distribution; particle morphology; and/or particle composition. In certain embodiments, the average size of particles that can be measured within the gas stream may range from 0.003 micrometer ($\mu$m) to 10 $\mu$m, or from .05 $\mu$m to 1 $\mu$m, or from 0.1 $\mu$m to 1 $\mu$m. In certain embodiments, the average amount of particles that can be measured may range from 1/ standard cubic foot ( scf) (35/standard cubic meter; "35/scm")to 10,000,000/ scf (280,000/scm), or from 1 / scf (35/scm)to 10,000/ scf (280/scm), or from 1 / scf (35/scm) to 1,000/ scf (28/scm).

The system and method can be used for a variety of gases and supercritical fluids including pyrophorics, flammables, oxidants, corrosives, and inert gases. Examples of gases that can be analyzed include, but are not limited to, inert gases (e.g., Ar, He, $N_2$, Xe, Kr, Ne), $SiH_4$, $CF_4$, $WF_6$, $SiH_2Cl_2$, $NH_3$, $NF_3$, $Cl_2$, $BCl_3$, $C_2F_6$, $CO_2$, CO, $F_2$, $N_2O$ $CHF_3$, $O_2$, $H_2$, HBr, HCl, HF, $CH_4$, $SiHCl_3$, $SF_6$, $PH_3$, $AsH_3$, $BF_3$, $B_2H_6$, $Si_2H_6$, $SiCl_4$, $SiF_4$, and many others, and mixtures thereof. The term "gas" encompasses vapors, supersaturated gases, and supercritical fluids. Non-limiting examples of particular supercritical fluids are provided in pending U.S. Published Application No. 2004/0144399. The system may be used, for example, to measure and/or analyze a variety of particulates which may include, for example, molecular clusters, liquid droplets, suspended solid particulates consisting of metallic, organic or other materials, and various other contaminating particles.

The system described herein measures and analyzes particles within a representative sample of a process gas stream by using a particle counter and/or a particle capture filter. The system preferably does not significantly alter the quantity of molecular impurities present in the gas. Such interferences could change the measured particle concentration in the gas stream being sampled. In certain embodiments, electropolished tubing and/or high cleanliness valves may be used to reduce sampling bias. Further, in these and other embodiments, the system minimizes transport losses of particles resulting from gravitational settling or diffusion to tube walls by reaction to molecular Brownian motion.

The system described herein may be used in conjunction with a continuous process feed stream or with side stream or sample stream extraction systems, described herein as "off-line sampling". The system described herein may also be used in conjunction with one of the gas sample extraction devices well known in the art, including so-called isokinetic sample probes, inserted into the feed gas line. In this connection, a separate stream to be sampled, which is referred to herein as the gas stream, is withdrawn from the process gas line, pressurized gas cylinder, or ISO module (i.e., an arrangement of cylindrical tubes in a single integrated unit suitable for bulk transport of gases). The off-line sample system may require venting or other form of emission control of the gas stream that is flowed through the particle quantification device (i.e., the particle counter and/or the particle capture filter). In embodiments where the gas stream comprises a reactive gas, the off-line sample system may further include sub-systems to provide inert purging (i.e., purging the gas line with one or more inert gases), evacuation (i.e., evacuating the gas line using one or more vacuum pumps), and/or emission control. In other embodiments, such as when the gas stream comprises easily condensable gases (i.e., gases that become liquids at a temperature at or above ambient), the system lines and/or system components contained therein may also be heat traced. In these embodiments, heat tracing may be used in combination with inert purging and/or pressure cycling (i.e., employing a pressure variation) prior to introduction of the gas stream into the line for initial system drying because the gas may react with trace residual moisture or oxidizers in the line.

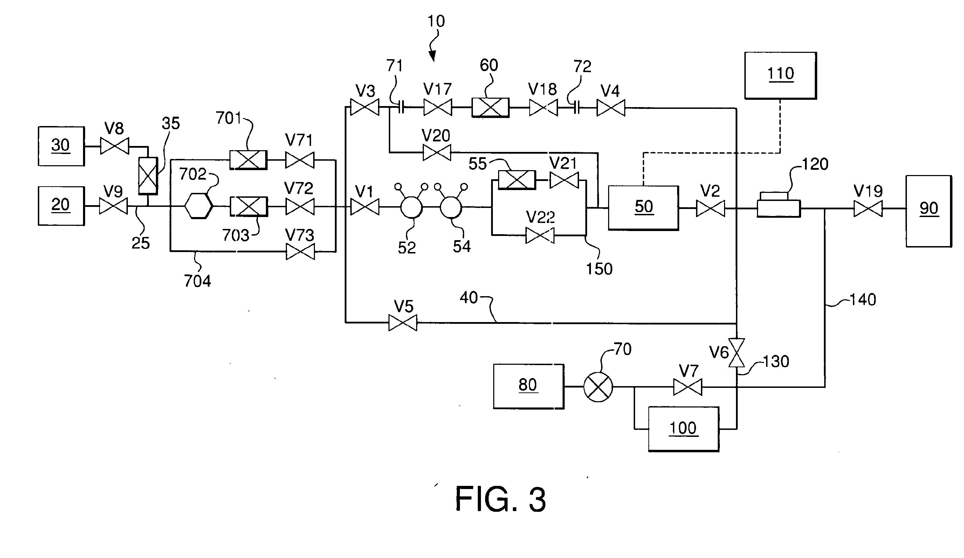

Figs. 2 and 3 provide examples of embodiments of an off-line sample system according to the present invention used to measure and analyze impurity generated particles within a gas. In certain embodiments, such as that depicted in Fig. 3, system 10 is housed within a ventilated enclosure and is at ambient temperature. The gas stream 25 may be provided from supply source 20 such as a storage tank, pressurized gas cylinder, transfill line, gas distribution line, or other means (not shown). Supply source 20 may be of large or small volume. At least a portion of the gas from supply source 20 is removed to provide a gas stream 25 which is fed through sampling system 10. Gas stream 25 is typically introduced into system 10 at a pressure ranging from 4 psia to 10,000 psig (27 kPa to 69 MPa), or from 0 psig to 3,000 psig (0 to 21 MPa), or from 100 psig to 1,600 psig (0.7to 11 MPa), depending upon, for example, the supply source volume, the identity of the gas stream 25, whether the gas contained therein is in a supercritical state, etc. System 10 further includes inert gas source 30 that is in fluid communication with system 10 and used for inert gas purging and other means. Inert gas purifier 35 is connected in fluid communication to inert gas source 30 to provide a purified inert gas for such purging/flushing operations.

In embodiments where gas feed stream 25 comprises silane, an inert gas purge followed by one or more evacuation cycles should be conducted prior to the introduction of the gas stream. Still referring to Fig. 3, an inert gas purge is conducted by closing valves V9 and V19 and opening valves V7 and V8. After the gas stream 25 has been analyzed, an inert gas purge may be run in a similar manner to remove any residual silane from the system. An evacuation cycle is conducted by closing valves V8, V9, and V19 and opening valve V7.

The embodiment depicted in Fig. 3 employs particle counter 50 and/or a particle capture filter 60 such as a membrane type filter to measure and detect the particles within the gas feed stream. Particle counter 50 can be connected to the gas source through a plurality of pressure regulators. Flow through the instrument is regulated by a flow control valve and a mass flow meter. Gas flows to the particle counter or particle capture filter through bypass line 704. In certain embodiments, the particle counter and/or particle capture filter operate(s) at a pressure of near atmospheric pressure (101 kPa). In these embodiments, pressure reduction of the incoming gas stream 25 may be required between supply source 20 and particle counter 50. In the system shown in Fig. 3, the pressure reduction is performed in two steps using two pressure regulators, 52 and 54, arranged in series. Such multi-step pressure reduction minimizes particle shedding and gas condensation which tends to occur during the pressure reduction process. In other embodiments of the invention, a single step pressure reduction may be used. In other embodiments, gas stream 25 going to particle counter 50 may not need to be reduced because its pressure is sufficiently low. After pressure reduction, the gas flows to the particle counter or particle capture filter through bypass line 150.

Some commercially available particle counters can be used for UHP process gas monitoring. These instruments are sometimes referred to as condensation nucleus counters (CNCs). CNCs can detect individual particles as small as 0.003 $\mu$m but provide no information on particle size distribution. A different type of instrument is sometimes referred to as an optical particle counter (OPC). Safe and chemically compatible instruments are available for use with silane. A non-limiting example of a suitable particle counter is manufactured by Particle Measuring Systems (PMS), Inc. of Boulder, CO. The PMS, Inc. model HPGP-101-C can detect particles having an equivalent optical scattering diameter as small as

0.1 $\mu$m or 100 nanometers (nm), with a counting efficiency of >50% at 140 nm using a HeNe laser. The instrument requires a sample flow rate of 0.1 standard cubic foot per minute (2.8 standard liters per minute). The HPGP-101-C has a zero count level of <2/ft$^3$ (<35/m$^3$), or <0.2/minute, and can measure particle concentrations up to 3,000/scf (100/l). The instrument has 8 size channels, with thresholds at 100, 200, 300, 500, 1000, 2000, 3000 and 5000 nm. The sampling interval can be set in the range of from 1 second to 100 hours.

When not in use, particle counter 50 can be isolated from the system by closing valves V1 and V2. This permits particle counter 50 to remain free of contaminants that may otherwise enter the system.

In preferred embodiments of the present invention, particle counter 50 is combined with particle capture filter 60 to provide a means of determining when spurious start-up induced counts are removed from the system. Such spurious counts result from valve actuation and/or reaction between reactive matrix gases and trace residual impurities in system 10. In such embodiments of the present invention, a gas stream can be simultaneously or sequentially directed to particle counter 50 and capture filter 60 for measurement. For example, gas stream 25 can be first directed to particle counter 50 and then a portion of gas stream 25 can be directed to capture filter 60 to corroborate the results observed by particle counter 50 and to further characterize the particles as explained in more detail below. In these embodiments, only then is the isolated capture filter exposed to the incoming gas stream 25.

Particle capture filter 60 is preferably located in a parallel leg of the sampling system relative to particle counter 50. Particle capture filter 60 can operate with or without pressure reduction; and the gas stream 25 can flow through valve V3 to particle capture filter 60 at full system pressure or through valve V20 to particle capture filter 60 at reduced pressures. This direct sampling method minimizes the potential for spurious particle counts resulting from "shedding" of pressure reducers. Capture filter 60 also permits examination of the captured contaminant particles under various analytical tools such as, but not limited to, scanning electron microscopy (SEM), energy dispersive X-ray spectrometry (EDS), light microscopy, and other means. This technique provides additional information on morphology and composition of impurity generated particles. Such information aids in identification and elimination of molecular impurity sources within the system.

In preferred embodiments of the present invention, capture filter 60 has two fittings 71, 72 that allow capture filter 60 to be removed from the system. Capture filter 60 can be readily removed at fittings 71, 72 when valves V3, V4, V17, V18 and V20 are closed.

Once removed from system 10, the particles collected on capture filter 60 can be analyzed by employing the following method. First, the background contamination (i.e., contamination that may be present on the surface of capture filter 60 before exposure to gas stream 25) on the surface of capture filter 60 is preferably distinguished from the sample contamination. Background contamination typically originates during the filter manufacturing and handling process. In this regard, the surface density of background contamination on filter 60 must be measured and accounted for in the particle capture method. Microscopy is used to determine the number of background particles on an un-exposed filter. This can be done by examining only a part of the filter surface. A portion of the filter's surface area, $A_B$, is inspected to obtain the number of background particles, $N_B$, in that area. After exposure to the sample gas or supercritical fluid a portion of the surface area, $A_P$, is inspected to determine the total number of background and captured particles, $N_P$, in that area. The total number of captured particles, N, on the entire surface of the exposed filter is then given as:

$$N = A \, (N_P/A_P - N_B/A_B),$$

where A is the total surface area of the filter. If V is the volume of sample gas or supercritical fluid passed through the exposed filter, then the concentration of particles per unit volume of sample, C, is given as:

$$C = N/V.$$

Sampling system 10 also has a bypass line 40 to permit cycle/purging of both sides of the particle filter 60, and to permit flow initialization around the filter 60. Bypass line 40 includes valve V5 that, when open, allows for cycle-purging of sampling system 10 with inert gas from inert gas source 30 by a downstream vacuum pump 70, such as, for example, a turbo-molecular vacuum pump. Vacuum pump 70 is in fluid communication with system 10, once valve V6 is open, through flow line 130.

In some embodiments of the present invention, vacuum pump 70 is used to draw sample fluids from low pressure sources. The sample fluid passes through the particle counter 50 or capture filter 60 before passing through vacuum pump 70 and then into emission control system 80. Emission control system 80 may comprise, for example, a gas reclamation system, a combustion system, a vent system, a scrubber system, an adsorption system, an absorption system, or a purification and storage system. Such systems are well-known in the art of vent stream emission control.

In certain embodiments of the present invention, capture filter 60 may be a track-etched filter or a porous alumina filter. Unlike automatic particle counters, capture filters have no upper limit on measurable particle concentration. Polycarbonate track-etched filter membranes are avaiiabie with pore sizes as small as 15 nm.

Alumina filter membranes are available with pore sizes as small as 20 nm. The higher pore density of alumina filters provides a minimal flow resistance at high flow rate. A high flow rate is beneficial in sampling a large volume of gas in a minimum time. The particle capture filter 60 may be housed within a pressure resistant filter housing such as, for example, a Model No. xx4502500 25 mm stainless steel filter housing manufactured by Millipore Corporation of Bedford, MA. The filter housing contains the filter membrane, which may be sealed therein with a variety of elastomeric materials, such as for example a PTFE O-ring. This filter membrane may be used, for example, to capture particles in various high-pressure or reduced pressure gases, including silane. The particles within the gas feed stream can be analyzed using a variety of techniques, such as, but not limited to, light microscopy, SEM and EDS, after being captured on the filter.

In preferred embodiments of the present invention, particle capture filter 60 may be a chemically resistant filter media, such as, for example, PTFE microporous membranes. Such membranes are not suitable for EDS or microscopic particle examination due to their rough surface structure. However, particles captured on such filters can be analyzed for composition and total captured mass by digestion or dissolution through immersion in various liquid acids or solvents. The acid or solvent is then analyzed through various well-known means, including liquid chromatography.

Sampling system 10 is designed for turnkey operation, and connection to any selected gas feed source. The system can be used for periodic cylinder qualification tests, point-by-point impurity survey studies of gas distribution systems, or continuous alarmed monitoring of a gas transfill system or a gas distribution system.

The pressure of incoming gas feed stream 25 in system 10 is typically measured by employing a pressure gauge, such as for example a diaphragm-type pressure gauge (not shown). In the system depicted in Fig. 3, the silane gas may be sent to an on-site burner system 90. However, depending upon the identity of the gas stream 25, the sample gas may alternatively be vented, reclaimed, or sent to emission control system 80 or an absorber, scrubber, purifier and storage system (not shown), or recycled back into the main gas supply (not shown).

Flow control device 120 is typically employed to control and monitor the rate of sample gas flow through the particle counter or the particle capture filter during testing. Flow control device 120 may include a manually operated flow control valve and a flow meter such as, for example, a mass flow meter, or flow control device 120 may include an automatically actuated flow control device such as, for example, a mass flow controller. Sample gas downstream from flow control device 120 can be evacuated through flow line 140, which is also in fluid communication with vacuum pump 70.

In preferred embodiments of the present invention, pneumatically actuated valves can isolate the clean internals of the system when it is not in use. The cycle-purging and sampling sequence can be performed automatically using a process logic controller (PLC) (not shown) before each sample run. The PLC receives input from a pressure transmitter and heat tracing temperature controller to ensure the vacuum- pressure cycle is within specified limits during operation. In certain embodiments, the heat traced lines are held at 100°C during cycle purging. The system is evacuated to <50 Torr (<6.7 kPa) and returned to atmospheric pressure (101 kPa) at least 150 times during cycle-purging.

The system is flushed with purified inert gas prior to cycle-purging to eliminate atmospheric gases, and after sampling to remove any residual gas. Depending upon the identity of the gas being sampled, the flush cycle may send these gases to a reclamation system, a burner, or scrubber vessels, such as, for example, when the sample gas comprises silane. In other embodiments, the flush cycle may vent the gas to atmosphere. The purge operation is performed while the system is connected to the gas source, before and after the sampling procedure. The sample source valve is closed during the inert gas flush operation. In embodiments where the gas being sampled comprises silane, this purge gas is sent to a silane burner, reclaim system, or scrubber. The inert flush process may also be used to purge-out the gas source connection fitting while the system is disconnected from the gas source. This purge is intended to prevent contaminants from entering the open sampling system. The inert gas will vent out the gas inlet line to atmosphere.

In preferred embodiments of the present invention, such as that depicted in Fig. 3, heat tracing should be included to minimize residual molecular impurities in the system 10. Such residual impurities would degrade the accuracy of the desired impurity measurement. In these embodiments, all system components upstream of the particle counter and capture filter are preferably heat traced. Heating tracing comprises electrical resistance heating elements that may be affixed, for example, to the outer surfaces of system tubing, valves, filter housings, pressure regulators, and other components. The heat tracing includes a temperature sensing device, such as, for example, a thermocouple to provide temperature feedback to a temperature indicator, and to a temperature control device such as, for example, a process controller or thermostat. The temperature control device contains circuitry designed to regulate the power to the heating elements such that a set temperature is maintained within the system. Such heating provides ready elimination of trace absorbed moisture from the internal surfaces of the system, and permits removal of trace residual sample fluids from the system following the testing process.

In certain embodiments, system 10 may employ a moisture analyzer 100 that can detect residual moisture in the line and may, for example, activate various valves if the level of moisture within the gas stream is outside desired levels. In this or other embodiments, system 10 may employ an oxygen sensor that acts in a similar fashion as the moisture analyzer in detecting the presence of oxygen within the gas stream.

In another preferred embodiment of the present invention, the method and system described herein may be used to remove molecular impurities and particles through the use of one or more purifying devices 702 and in-line filters 703 from a gas stream at a higher pressure to provide a purified and filtered gas stream, and to reduce the pressure of the purified and filtered gas stream to a lower pressure gas stream without consequent induced particle formation. In this embodiment, gas bypass line valve V73 is closed and purifier/filter line valve V72 is opened. In this embodiment of the invention, the "background" level of particles measured by particle counter 50 and/or particle capture filter 60 can be obtained, unimpeded by impurity generated particle formation. Such background levels are then subtracted from later measurements obtained by flowing the gas through bypass line 704.

The term "lower pressure gas feed stream" as used herein describes a purified gas feed stream that has been passed through a pressure reducing device, such as without limitation, an automatic pressure regulator, valve, flow restricting orifice, or the like. The pressure of the initial gas feed stream is reduced to a level compatible with the available instrumentation for particle measurement after purification. For example, in one embodiment of the present invention, the gas feed stream may be at an initial pressure ranging from 150 (1.1 MPa) to 10,000 psig (69 MPa). A pressure reducing device then reduces the pressure of a purified gas feed stream to a pressure ranging from 0 to 150 psig (0 to 1.1 MPa). The pressure ranges described herein may vary depending upon the initial pressure of the gas feed stream, the type of pressure reducing device used, the particle measurement device, and/or other variables.

The term "purified gas feed stream" as used herein describes a gas feed stream that has been passed through one or more purifying devices to remove various impurities contained therein. The system and method described does not adversely affect (such as increase) the particle content of the gas feed stream that may be attributable to pressure reducing devices.

A purifying device as used herein is a device that reduces the level of the impurities contained within the gas feed stream without adversely affecting the chemical composition of the gas.

In preferred embodiments of the present invention, the purifying medium within the purifying device may consist of various well-known adsorbent, absorbent or catalytic materials, such as activated carbon, desiccants (e.g., Drierite™), phenolic resins (e.g., Ambersorb™), nickel catalyst, copper catalyst, etc., which are selected depending upon the type of impurities to be removed and/or the gas feed stream composition. In one preferred embodiment of the system described herein, a purifying medium consisting of Ambersorb™ pellets is contained in a vessel to remove certain hydrocarbons and siloxanes from silane gas. In another preferred embodiment of the present invention, a purifying medium consisting of granular activated carbon is contained in a vessel to remove certain hydrocarbons and water from nitrogen gas.

In certain embodiments, the system and method described herein may allow for replacement or regeneration of the purifying medium. Regeneration may be accomplished using methods well-known in the art of gas purification, such as, but not limited to, exposure to high purity inert regeneration gas at elevated temperatures. Replacement is accomplished by removal of the purifying material, or of an entire purifier device from the gas line.

In yet another embodiment, the purifying device may comprise cryogenic cold trapping of higher condensation point/freezing point impurities to remove impurities prior to pressure reduction. In one preferred embodiment of the invention, cryogenic cold trapping is used to remove certain higher molecular weight hydrocarbons and water from nitrogen gas.

The purified gas stream is then passed through a pressure reducing device to provide a lower pressure gas stream. The lower pressure gas stream is then sent to a particle counter and/or a particle capture filter.

In another preferred embodiment of the present invention, the method and system described herein comprises one or more in-line filters 701 to remove particles from a gas stream at a higher pressure to provide a filtered gas stream, and to reduce the pressure of the filtered gas stream to a lower pressure gas stream. In this embodiment, gas bypass line valve V73 is closed and filter line valve V71 is opened. In this embodiment of the invention, the level of impurity generated particles formed during pressure reduction can measured by particle counter 50 and/or particle capture filter 60, unimpeded by suspended particles from gas source 20. Such "background" levels are then subtracted from later measurements obtained by flowing the gas through bypass line 704.

In another preferred embodiment of the present invention, the method and system described herein may be used to remove particles through the use of one or more in-line gas filters 55 from a gas stream at a lower pressure to provide a filtered gas stream. In this embodiment, gas bypass line valve V22 is closed and filter line valve V21 is opened. In this embodiment of the invention, the "background" level of particles measured by particle counter 50 and/or particle capture filter 60 can be obtained, unimpeded by impurity generated particle formation, "shedding" generated particle formation and suspended particles from gas source 20. Such background levels are then subtracted from later measurements obtained by flowing the gas through bypass line 150.

The total mass of particles detected by the particle counter and/or particle capture filter is used to determine the original concentration of molecular impurity present in the UHP gas. The invention utilizes a predetermined calibration curve to relate the mass of particles to the molecular impurity level entering from the UHP gas source 20. This invention can serve as an early warning process monitor to detect extremely low levels of contaminant impurities present in the system. Typically, the particle detector may be connected to a process alarm in order to alert operators of particle levels exceeding a set level.

Referring to the preferred embodiment of Fig. 2, UHP gas passes through filter 16 as it enters UHP gas system 20. Modern micro-porous process gas filters have extremely high retention efficiencies, and can effectively eliminate all particles entering a UHP gas system. All other sources of particles downstream of the filter are eliminated or minimized in their effect on the gas-borne particle level. Examples of such sources include valves, dead leg regions, and other generators of particulate matter. Elimination of such particle sources is routinely done in UHP gas systems. Any particles present downstream of the filter are therefore a result of reaction with impurities entering through the filter, or through reactive impurities sources located downstream of the filter.

Test system 10 is set to extract a gas sample from an appropriate monitoring point located downstream of filter 16, and preferably close to the sensitive IC fabrication tools 200. Examples of fabrication tools utilizing this inventive method for impurities monitoring include but are not limited to any of the various chemical vapor deposition (CVD) epitaxial deposition tools manufactured by Applied Materials, Inc. of Santa Clara, CA, USA.

A preferred means for extracting sample gas for the purpose of measuring the suspended particle level is disclosed in U.S. Provisional Patent App. Serial No. 60/649,490, filed February 3, 2005, and entitled "System and Method Comprising Same for Measurement and/or Analysis of Particles in Gas Stream" and the corresponding non-provisional application filed January 27, 2006 as Application Serial No. 11/340,641 (now US-A-2006/0172428; corresponding to EP-A-1688731).

This inventive method for detecting particle generating impurities in a gas can be extremely sensitive. For example, the LDL of one silane-compatible automatic particle counter is 0.1 micrometer. This represents an optical diameter equivalent to the instrument's aerosol calibration standard. The instrument was calibrated using polystyrene latex micro-spheres of a known size suspended in nitrogen. The sensitivity of this invention is limited by the instrument-generated noise level of the OPC. The noise level is produced by electrical effects or cosmic rays resulting in spurious electrical pulses in the instrument's discriminator circuit. One commercially available instrument has a noise level of less than 2 counts per standard cubic foot (less than 70 counts per scm) of sample gas.

Assuming a signal to noise value of no less than one to practice certain preferred embodiments of the invention, then the lowest theoretical sensitivity of these embodiments can be calculated as follows:

- A volume of one cubic foot (28 liters) contains 1.18 gm moles of silane under standard conditions.

- Two 0.1 micrometer diameter spherical particles contain a total of $3.24 \times 10^{16}$ gm moles of silica (natural cristabolite, density = 2.32 gm/cm3)

- Therefore, two 0.1 micrometer diameter silica particles in 1 standard cubic foot (28 NL) of silane correspond to:

- $(3.24 \times 10^{-16})/1.18$ mole/mole = $2.75 \times 10^{-16}$ mole/mole oxygen in silane, or

- $2 \times 2.75 \times 10^{-16}$ mole/mole = $5.48 \times 10^{-16}$ mole/mole water in silane.

This is the lowest theoretical resolution for oxygen or water using this preferred embodiment of the invention. This is much better resolution than for conventional impurities monitors such as APIMS. The actual limit of detection of the invention for a given gas at a given initial pressure depends upon the minimum concentration of impurity necessary to initiate reaction and/or nucleation into suspended particulate matter, and is determined through a calibration process.

In embodiments of the invention comprising a calibration step, a preferred calibration curve would relate the measured total mass of reaction-induced or nucleation-induced particles detected to the total mass of impurities in the gas prior to particle formation. Preferably, such a calibration curve will show a monotonic increase of particle mass with impurities mass for a given matrix gas at a given initial pressure, and a given reduced pressure.

OPCs require relatively little effort to re-calibrate, and are inexpensive to purchase, operate and maintain. They also occupy little space and can operate unattended for extended periods of time. Calibration checking requires only filtered (particle free) gas, and a suitable aerosol standard. The instrument is typically checked for detection limit off-line using standard polystyrene latex micro-spheres suspended in an inert gas stream. Sensitivity is checked using filtered inert gas. Such gases are easy and safe to produce. This re-certification procedure is simple compared to that of some molecular impurities detectors, such as APIMS.

Referring again to Fig. 3, system 10 may employ a computer 110 that is in electrical communication with, for example, particle counter 50, the PLC, or other system components. Computer 110 can operate certain valves (not shown) within the UHP gas system 20 to automate the system based upon certain parameters within the gas stream ( e.g., particle concentration, pressure, temperature, moisture content, oxygen content, etc.). In preferred embodiments, system 10 may also employ a sensor (not shown) that measures the amount of particulate within the gas stream and a controller (not shown) that is in electrical communication with the sensor such that if the sensor measures the amount of particulate at a point that is above a set point, then the sensor directs the UHP gas system 20 valves to close.

A preferred method for measuring particle content within a gas stream according to the present invention comprises the steps of passing at least a portion of a gas stream through a filtration device to provide a filtered gas stream wherein the filtration device does not substantially remove the molecular impurities contained within the filtered gas stream, wherein the gas stream is at a first pressure. Next, for example, a portion of the filtered gas stream can be directed to a pressure reducing device to reduce the pressure of the portion of filtered gas stream to a pressure that is lower than the first pressure. Next, the particle content contained within the filtered gas stream is measured by passing the portion of filtered gas feed stream that is at a pressure lower than the first pressure to a particle counter and by passing another portion of the filtered gas stream to a particle capture filter, wherein the particle capture filter is arranged in parallel with the particle counter.

This invention extends the available LDL for impurities in silane. The method is useful for any impurity that reacts or nucleates to form suspended particles.

As noted above, the invention can be applied to other systems in which a reactive or nucleating gas reacts or nucleates to form suspended particles in the gas. Non-limiting examples include certain hydrocarbon impurities in nitrogen or oxygen impurity in $SiH_2Cl_2$.

EXAMPLES

Additional objects, advantages, and novel features of this invention will become apparent to those skilled in the art upon examination of the following examples thereof, which are not intended to be limiting.

Example 1. Test system 10 was purged with inert gas, heated to 50°C and cycle purged for an extended period. Purified and filtered helium at a pressure of 1,700 psig (11.8 MPa) was then sampled from a UHP gas system 20 using test system 10. The helium was passed at high pressure through bypass line 704 and reduced in pressure to 80 psig (650 kPa) through single regulator 52. The reduced pressure helium then flowed through bypass line 150 into particle counter 50, which consisted of a PMS, Inc. model HPGP-101 optical particle counter. The particle counter indicated a particle concentration in the reduced pressure helium of 300/ scf (11/l) for particles 0.1 micrometer in size. A volume of one cubic foot (28 liters) contains 1.17 gm moles of helium under standard conditions. The non-volatile impurity in this example is taken to have a liquid density of 0.77 gm/cm³ and a molecular weight of 226.45 gm/gm mole. Three hundred 0.1 micrometer diameter spherical particles contain $5.34 \times 10^{-16}$ gm moles of impurity. Therefore, three hundred 0.1 micrometer diameter impurity particles in 1 standard cubic foot (28 NL) of helium correspond to

$4.85 \times 10^{-16}$ gm mole/gm mole of impurity in helium. Therefore, the impurity in helium was present at a level of at least $4.85 \times 10^{-14}$ molar percent.

Example 2. Test system 10 was purged with inert gas, heated to 50°C and cycle purged for an extended period. Un-purified and un-filtered silane at a pressure of 1,000 psig (7 MPa) was then sampled from a gas cylinder 20 using test system 10. The silane was passed at high pressure through bypass line 704 and reduced in pressure to 80 psig (650 kPa) through single regulator 52. The reduced pressure silane then flowed through in-line filter 55 into particle counter 50, which consisted of a PMS, Inc. model HPGP-101 optical particle counter. The particle counter indicated a particle concentration in the reduced pressure silane of approximately 0/ scf (0/scm). This result demonstrates a low background count rate for the test system 10 when sampling reactive silane gas.

Example 3. Test system 10 was purged with inert gas, heated to 50°C and cycle purged for an extended period. The same un-purified and un-filtered silane at a pressure of 1,000 psig (7,000 kPa) was then sampled from a gas cylinder 20 using test system 10. The silane was passed at high pressure through bypass line 704 and reduced in pressure to 80 psig (650 kPa) through single regulator 52. The reduced pressure silane then flowed through bypass line 150 into particle counter 50, which consisted of a PMS, Inc. model HPGP-101 optical particle counter. The particle counter indicated a particle concentration in the reduced pressure silane of 500,000/ scf (17,900/l) for particles 0.1 micrometer in size. This measured particle concentration did not change when the silane was passed at high pressure through in-line filter 701. Therefore, the measured particles were formed during pressure reduction in single regulator 52. A volume of one cubic foot (28 NL) contains 1.18 gm moles of silane under standard conditions. Since the measured particles formed by nucleation not reaction, the impurity in this example is taken to be a non-volatile impurity having a liquid density of 0.77 gm/cm$^3$ and a molecular weight of 226.45 gm/gm mole. Five hundred thousand 0.1 micrometer diameter spherical particles contain $8.91 \times 10^{-13}$ gm moles of impurity. Therefore, five hundred thousand 0.1 micrometer diameter particles in 1 standard cubic foot (28 NL) of silane correspond to $7.55 \times 10^{-13}$ gm mole/gm mole of impurity in silane. Therefore, the impurity in silane was present at a level of at least $7.55 \times 10^{-11}$ molar percent.

While the invention has been described in detail and with reference to specific examples thereof, it will be apparent to one skilled in the art that various changes and modifications can be made therein without departing from the scope thereof.

**Claims**

1. A manufacturing process comprising supplying to a processing apparatus a gas containing at least one impurity that reacts and/or nucleates to form contaminating particles suspended in the gas, wherein a particle counter and/or particle capture filter samples a flow of the gas upstream of the processing apparatus to detect an amount of the contaminating particles suspended in the flow of the gas, and a signal is generated when the amount of the contaminating particles is in excess of a predetermined amount.

2. The process of claim 1, wherein the processing apparatus is a chemical vapor deposition device.

3. The process of claim 2, wherein the processing apparatus is a chemical vapor deposition epitaxial deposition tool.

4. The process of any preceding claim, wherein the gas is silane.

5. The process of any preceding claim, wherein the contaminating particles are products of reactions with at least one of water and oxygen.

6. The process of any preceding claim, wherein the contaminating particles are nucleated molecular impurities formed by pressure reduction.

7. The process of any preceding claim, wherein the gas is silane and the contaminating particles comprise at least one member selected from siloxane particles and silica particles.

8. The process of claim 7, wherein a maximum acceptable concentration of silica is determined from the predetermined amount using a calibration curve.

9. The process of claim 7 or 8, wherein the predetermined amount of particles correlates with a concentration of $2.75 \times 10^{-6}$ mole silica per mole silane in the gas stream.

10. The process of claim 7 or 8, wherein the predetermined amount of particles correlates with a silica concentration of 10 parts per trillion.

11. The process of any preceding claim, wherein the particle counter is an optical particle counter.

12. The process of any preceding claim, wherein the flow of the gas into the processing apparatus is interrupted when the signal is generated.

13. The process of any preceding claim, wherein the signal comprises an audible alarm.

14. The process of any preceding claim, comprising determining at least one of: (a) a number of particles; (b) a concentration density of particles; (c) a particle size distribution; (d) a particle morphology; and (e) a particle composition.

15. The process of any preceding claim, wherein both the particle counter and the particle capture filter sample the flow of the gas upstream of the processing apparatus to detect the amount of the contaminating particles.

16. The process of any preceding claim, wherein an average size of the contaminating particles is from 0.003 $\mu$m to 10 $\mu$m.

17. The process of any preceding claim, wherein an average amount of the contaminating particles measured is from 35/standard cubic meter to 280,000/standard cubic meter (1/standard cubic foot to 10,000,000/standard cubic foot).

18. An integrated circuit manufacturing process comprising supplying gaseous silane to a processing apparatus, wherein at least one of a particle counter and a particle capture filter samples a flow of the silane upstream of the processing apparatus to detect an amount of contaminating particles suspended in the flow of the silane, and a signal is generated when the amount of contaminating particles is in excess of a predetermined amount.

19. The process of claim 18, wherein the process is as further defined in one or more of claims 2, 3, or 5 to 17.

20. An apparatus for maintaining a purity level of a gas feed stream of a processing apparatus at or above a minimum acceptable purity level, said apparatus comprising:

> a supply source (20) for supplying the gas;
> at least one processing tool (200) in fluid communication with the supply source and adapted to use the gas to perform a processing function;
> a particle counter (50) placed upstream of the processing tool and downstream of the supply source;
> a microprocessor (110) in electrical communication with at least the particle counter; and optionally
> a particle capture filter (60) arranged in parallel with the particle counter upstream of the processing tool and downstream of the supply source.

21. The apparatus of claim 20, further comprising at least one valve controlled by the microprocessor such that the gas feed stream to the at least one processing tool can be terminated when the microprocessor determines that an amount of contaminating particles in the gas feed steam exceeds a predetermined value.

22. The apparatus of claim 20 or 21, comprising both the particle counter and the particle capture filter.

23. The apparatus of claim 20, 21 or 22, wherein the processing tool, gas, and/or particle counter are as further defined in one or more of claims 2, 3, 4, and 11.

# FIG. 1

PRIOR ART

Impurities

impurities

UHP Gas

16

20

200

25

10

# FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040144399 A
- US 64949005 P
- US 11340641 B
- US 20060172428 A
- EP 1688731 A

**Non-patent literature cited in the description**

- **WEN et al.** Nucleation of Trace Amounts of Condensible Vapors in an Expanding Gas Jet. *J. Aerosol Sci.,* 1988, vol. 19 (1), 153-156